# EUROPÄISCHE PATENTSCHRIFT

(11) **EP 0 890 365 B1**
(45) Veröffentlichungstag und Bekanntmachung des Hinweises auf die Patenterteilung: **14.05.2003**
(21) Anmeldenummer: 97810462.8
(22) Anmeldetag: 11.07.1997
(51) Int. Cl.: A61L 9/12

(54) **Verdunstungs-Dispenser**
Evaporative liquid dispenser
Diffuseur de liquide par évaporation

(43) Veröffentlichungstag der Anmeldung: 13.01.1999
(73) Patentinhaber: HTS International Trading AG, CH-6340 Baar (CH)
(72) Erfinder: Ehrensperger, Markus, Dr., 8442 Hettlingen (CH); Studer, Hans-Jörg, 8335 Hittnau (CH)
(74) Vertreter: Frauenknecht, Alois J.

(56) Entgegenhaltungen:
- EP-A- 0 645 148
- WO-A-80/00792
- GB-A- 2 222 775
- US-A- 4 078 891
- US-A- 4 780 253

## Beschreibung

Die vorliegende Erfindung bezieht sich auf ein Verfahren gemäss dem Oberbegriff des Patentanspruchs 1 und auf einen Verdunstungs-Dispenser zur Durchführung des Verfahrens.

Grundsätzlich lassen sich desodorierende Spender (auch Luftverbesserer genannt) in vier Kategorien einteilen: In solche mit natürlicher Konvektion des Wirkstoffes (engl. air freshening material), in solche mit mechanischer Unterstützung der Zirkulation, in solche mit thermisch verstärkter Verdunstung der flüchtigen Komponenten des Stoffs und in Raumsprayspender, welche über eine schlagartig betätigte Pumpe eine feine Tröpfchenwolke verbreiten (EP -A1- 0 127 573 und WO 94/04280).

Diese letztgenannten Spender versprühen kurzzeitig eine intensive Duftwolke und neigen in kleinen Räumen, aufgrund ihrer Montage an oder bei einer Türe, dazu die einen Raum betretenden oder verlassenden Personen zu parfümieren.

Im Gegensatz hierzu verwendet eine andere Vorrichtung (WO 80/ 00792) einen kontinuierlich betriebenen, batteriegespeisten Ventilator, der auf die Oberfläche eines flüssigen Wirkstoffs bläst und diesen gleichmässig verdunstet. Das hierzu nötige konstante Niveau der belüfteten Flüssigkeitsoberfläche wird durch eine nach Art einer Vogeltränke angeordnete Flüssigkeitszufuhr - über eine mit ihrem Ausfluss in die Flüssigkeit eintauchende Flasche - erzielt.

Nachteilig bei dieser Vorrichtung ist der konstante Verbrauch an Wirkstoff, insbesondere beim Einsatz in nur zeitweilig aufgesuchten Wasch- und/oder Toilettenräumen. - Dies kann, beispielsweise bei Toiletten in Bürogebäuden, während den relativ grossen Zeitintervallen ohne Benutzung, ebenfalls zu einem "überparfümieren" führen und erfordert zudem einen unverhältnismässig häufigen, unwirtschaftlichen Austausch der Nachfüll-Flaschen mit Wirkstoff. Entsprechend gross ist auch der Batterieverbrauch; die notwendigen Service-Intervalle sind daher sehr kurz (einige Tage!).

Ausserdem entstehen Hygiene-Probleme; durch den ständig laufenden Ventilator erfolgt ein grosser, kontinuierlicher Eintrag an Keimen und dgl. in die offene Flüssigkeitsoberfläche, was zu sichtbaren Verkrustungen im offenen Behälter führt.

Die US -A- 4,078,891 beschreibt einen Luftreiniger, welcher am Stromnetz angeschlossen ist. Die zu reinigende Luft wird durch einen Ventilator über ein Filter angesogen und im Inneren des Gehäuses mit Wirkstoffen angereichert und wieder ausgeblasen. Diese Anreicherung erfolgt über in Flaschen eingetauchte Dochte und/oder Verdunstungstabletten, welche auf einem Gitter gelagert sind. Unterhalb des Gitters befindet sich der relativ grosse, netzbetriebene Ventilator sowie eine zugehörige Steuerelektronik, welche diesen vorgegeben, sequentiell ein- und ausschaltet.

Nachteilig sind bei dieser Anordnung, dass keine bedarfsabhängige Steuerung möglich ist, weil die inheränt geringe Verdunstungsrate nur kurze Stillstandszeiten erlaubt und daher ein Netzanschluss notwendig ist, der in Nassräumen, wie Toiletten etc. ein Sicherheitsrisiko darstellt und besondere teure Installationen erfordert. Das Innere des Gehäuses wird zudem, auch bei ausgeschaltetem Ventilator über das Filter und die Ausströmleitung von Aussenluft durchströmt, so dass sich der dort enthaltene Wirkstoff laufend unkontrolliert abbaut.

Es ist daher Aufgabe der vorliegenden Erfindung eine hygienische Vorrichtung zu schaffen, welche eine genau den Bedürfnissen der Benutzer anpassbare Wirkung erzielt, jedes Besprühen von Personen vermeidet und zudem wirtschaftlich im Betrieb und einfach zu installieren und bequem im Unterhalt ist.

Im weiteren soll die Vorrichtung möglichst universell einsetzbar und insbesondere für Toiletten-, Pissoir- und Waschräume geeignet sein. Die Lärmentwicklung der Vorrichtung soll minimal sein, so dass diese problemlos auch in anderen Räumen Verwendung finden kann, wie beispielsweise Warteräumen, Aufzügen, Telefonkabinen etc.

Diese Aufgabe wird nach den Merkmalen des Anspruchs 1 gelöst.

Das im Patentanspruch charakterisierte Verfahren nützt die natürliche - temperaturabhängige - Verdunstungsleistung der Wirkstoffe in einem quasi geschlossenen Verdunstungs-Raum, welcher je nach Bedürfnis, bzw. Benutzung der Umgebung und ihrer Infrastruktur (begehbarer Raum; Toilette etc.) kurzzeitig "gelüftet" wird, d.h. dessen mit Wirkstoff gesättigte Atmosphäre durch Aussen- oder Umgebungsluft ersetzt wird. Dies erfordert nur ein Minimum an Hilfsenergie und gewährleistet einen nahezu geräuschlosen Kurzzeit-Betrieb.

In weiteren abhängigen Ansprüchen sind vorteilhafte Weiterbildungen des Erfindungsgegenstands beschrieben.

Bewährt hat sich ein Zug-/Druckventilator nach Anspruch 2, welcher energetisch günstig ist und ein gerichtetes Durchspühlen des Verdunstungs-Raums bewirkt. Zusätzlich wirken die beiden strömungstechnisch voneinander getrennten Zug- und Druckkammern bei stillstehendem Laufrad als Labyrinth-Dichtungen und verhindern einen unkontrollierten Austausch der mit Wirkstoff gesättigten Atmosphäre an die Umgebung.

Zwei gemäss Anspruch 3 angeordnete Vorratsflaschen mit entsprechenden Verdunstungselementen, ergeben einen unterbruchsfreien Betrieb, da während den üblichen Serviceintervallen sich zuerst eine Flasche leert und die zweite noch einen Vorrat an Wirkstoff enthält, bevor man sie an die Stelle der ersten Flasche plaziert und an ihren Platz eine neue, volle Ersatz-Flasche einsetzt.

Die Verdunstungselemente sind hygienisch einwandfrei und lassen sich problemlos entsorgen; die Vorratsflaschen sind wiederverwendbar.

Durch den Einbezug von Blenden bzw. Schiebern, Ansprüche 4 bzw. 6, lässt sich das zulässige oder gewünschte austretende Volumen an verdunstetem Wirkstoff, beispielsweise abhängig von der Umgebungstemperatur und Raumgrösse einstellen. Dies ergibt eine Anpassung der "Grundbeduftung", welche grundsätzlich bereits durch die Leckage (Undichtigkeit) am stillstehenden Laufrad des Ventilators entsteht.

Ein oberhalb des Gerätegehäuses angeordneter Lufteinlass ist strömungstechnisch und in Bezug auf Staubeinwirkungen günstig, vgl. Anspruch 5.

Der Dispenser nach Anspruch 7 erfordert eine nur minimale Hilfsenergie und lässt sich an jedem beliebigen Ort leicht installieren.

Die Ausführungsform nach Anspruch 8 erfordert ein Minimum an Wartung, insbesondere beim Einbezug eines Solarmoduls.

Die Aktivierung des Ventilators gemäss Anspruch 9 ergibt eine Optimierung der Betriebsdauer bzw. von dessen Laufzeit.

Durch einen lichtsensitiven Schalter, Anspruch 10, lässt sich die Gesamt-Laufzeit des Ventilators nochmals reduzieren; zusätzlich können der Schallsensor (z.B. Mikrofon mit Ruhestrom) und der nachgeschaltete Verstärkerteil, zur Energieeinsparung, abgeschaltet werden.

Die Ausführungsform nach Anspruch 11 gewährleistet auch bei schlechten Lichtverhältnissen einen einwandfreien Betrieb. Bewährt haben sich hierfür amorphe Si-Zellen, die auch diffuses Licht zu nutzen vermögen.

Besonders vorteilhaft ist die bedarfsorientierte Ausgestaltung der Vorrichtung nach Anspruch 12. Als Detektionsgrössen gelten, in Toiletten- und Waschräumen, insbesondere das Schliessen einer Türe und/oder die Betätigung einer Wasserspülung.

Nachfolgend werden anhand von Zeichnungen Ausführungsbeispiele des Erfindungsgegenstands beschrieben.

Es zeigen:
- Fig. 1: einen Verdunstungs-Dispenser mit abgenommenem Gehäusedeckel und seinem Zug-/Druckventilator, in einer Schrägansicht von oben,
- Fig. 2: eine Darstellung des Dispensers nach Fig. 1, mit herausgenommenen Vorratsflaschen, und weiteren Einzelheiten, in einer Schrägansicht von unten,
- Fig. 3: das Laufrad des Ventilators Fig. 1 und 2,
- Fig. 4: einen Gehäusedeckel mit Lufteinlass und Ausblasund Austrittsöffnung und einem integrierten Solarmodul und
- Fig. 5: das Prinzipschaltbild eines die Raumbenutzung detektierenden Mikrofons mit Verstärkerschaltung.

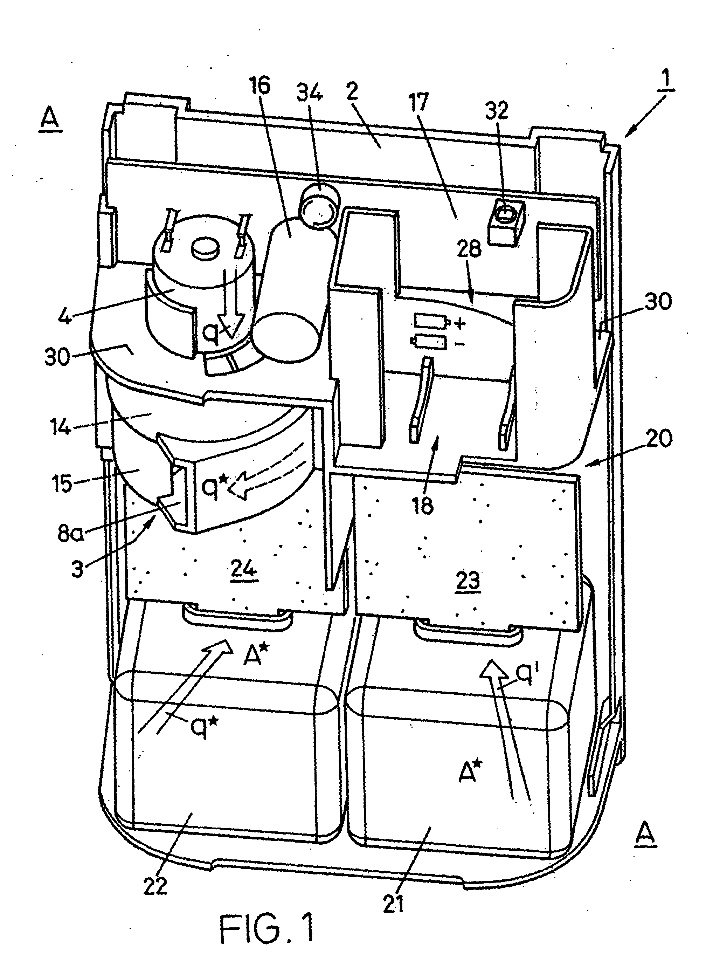

In Fig. 1 ist mit 1 Verdunstungs-Dispenser - eine Vorrichtung zum bedarfgerechten verdunsten von Wirkstoff - bezeichnet. Die Darstellung Fig. 1 ist mit abgenommenen Gehäusedeckel gezeichnet und daher in dieser Form nicht gebrauchsfähig.

Der Dispenser 1 basiert auf einer Montageplatte 2, welche bevorzugter Weise an einer Wand fixiert ist. Im oberen linken Quadranten befindet sich ein Verdichter 3, ein Zug-/Druckventilator mit zwei Kammern. Oberseitig am Verdichter 3 ist dessen Antrieb 4, ein Miniatur-Gleichstrommotor zu sehen.

Im unteren Teil des Verdichters 3 ist ein Volumenstrom q* eingezeichnet, welcher aus dessen unterer Kammer 15 eine gesättigte Atmosphäre A* in Richtung Ausblasöffnung 8a fördert.

Neben dem Antrieb 4 befindet sich ein Speicherkondensator 16, welcher bei der später beschriebenen Variante mit Solarmodul die Funktion eines Pufferspeichers übernimmt. Im oberen Teil der Montageplatte 2 ist, parallel zu dieser eine elektronische Schaltungsplatte 17 angeordnet, die u.a. einen lichtsensitiven Schalter 32 trägt. Unter diesem Schalter 32 befindet sich ein Batteriefach 18 zur Aufnahme von zwei handelsüblichen Monozellen, welche wie an der Rückwand symbolisch angedeutet miteinander in Serie geschaltet sind und somit 3 V ergeben.

Hinter dem Batteriefach 18 ist ein Zuluftkanal 28 angedeutet, der die obere Kammer des Verdichters 3 mit dem Verdunstungs-Raum 20 verbindet.

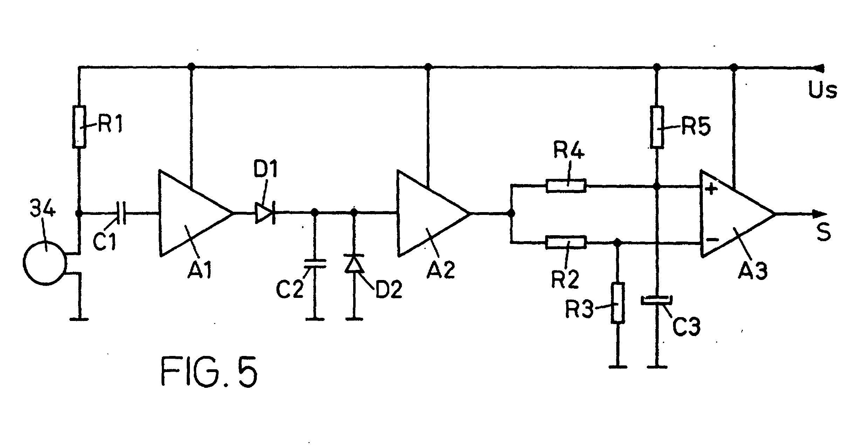

Sobald ein Gehäusedeckel 25, vgl. Fig. 4, auf die Montageplatte 2 aufgestülpt ist, bildet sich im innern des Dispensers 1 ein Verdunstungs-Raum 20, der über Verdunstungselemente 23, 24 und Vorratsflaschen 21, 22 laufend mit einem handelsüblichen Wirkstoff beschickt wird und somit die vorgängig erwähnte gesättigte Atmosphäre A* einschliesst.

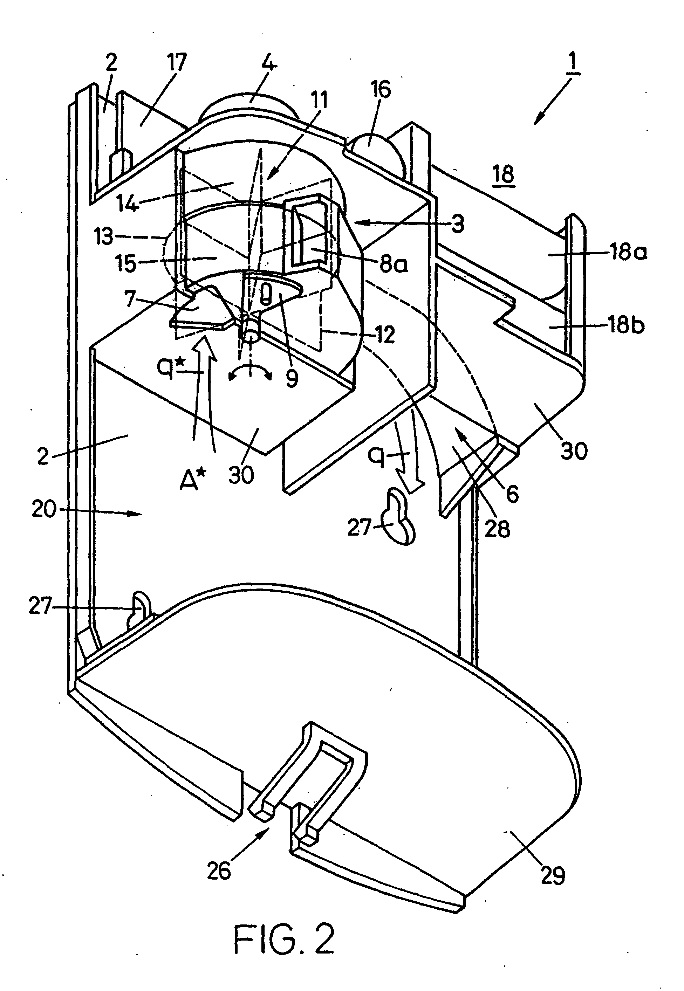

Der Dispenser 1 ist mit weiteren Einzelheiten in Fig. 2 dargestellt.

Hier ist u.a. ersichtlich wie der Verdichter 3 aufgebaut ist und wie das darin befindliche einzige Laufrad 11 mit seinen Schaufeln 12 in Kammern 14 und 15 angeordnet ist.

Die notwendige Trennung zwischen dem Zug- und dem Druckteil erfolgt am Laufrad 11 durch eine radiale Trennwand 13 welche ebenfalls angedeutet ist.

Unterhalb der Kammer 15 ist eine Absaugöffnung 7 für die angereicherte Atmosphäre vorgesehen, wobei die Absaugöffnung mittels einer Blende 9, an einem Hebel in Pfeilrichtung drehbar, in einfachster Weise eine Einstellung des Volumenstroms q* zulässt. Der Weg des Volumenstroms q mit der zugeführten Luft in den Raum 20 ist ebenfalls ersichtlich; er wird durch das Druckteil des Verdichters 3, über dessen oben partiell offene Kammer 14 über dem Kanal 28 angesaugt und an der Zuluft-Führung 6 ausgeblasen, was durch den Pfeil q angedeutet ist.

Im weiteren sind hier zwei Batterien 18a und 18b eingezeichnet; ebenfalls sind die beiden diagonal zu einander angeordneten Langlöcher 27, welche der Befestigung des Dispensers dienen in der Montageplatte 2 zu sehen. Unterhalb eines die Vorratsflaschen 21, 22 tragenden Supports 29 befindet sich eine Deckel-Verriegelung 26, die bei aufgesetztem Gehäusedeckel 25 in diesem einrastet.

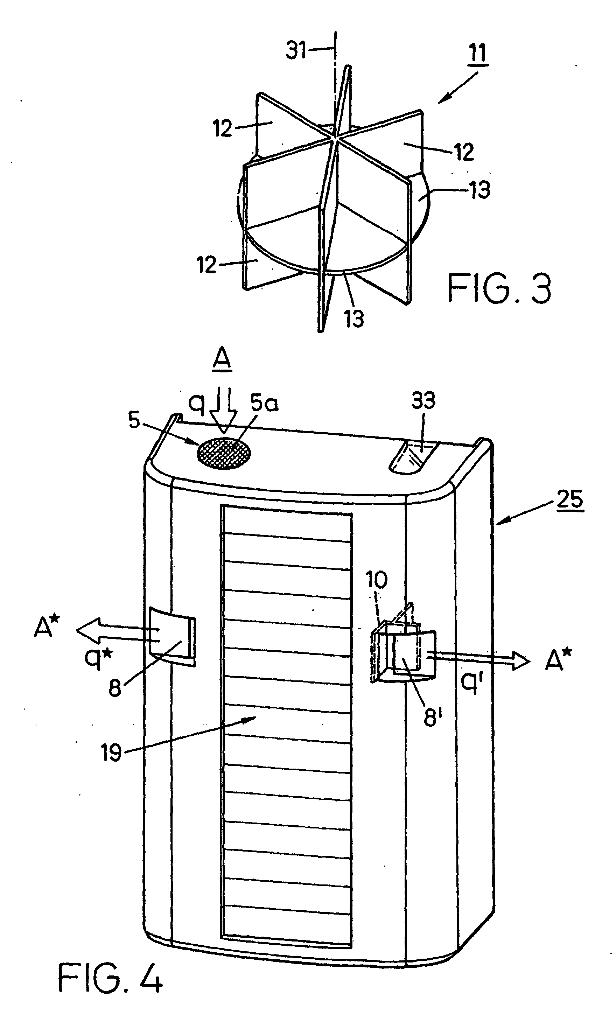

Im einzelnen ist das Laufrad 11 in Fig. 3 aufgezeigt. Wiederum ist die radiale Trennwand 13 zu sehen, welche die Schaufeln 12 in zwei Kammern teilen. Die Drehachse 31 ist lediglich angedeutet, sie ist die Motorachse des Antriebs 4, welche direkt in das Laufrad 11 eingesetzt ist.

Der Gehäusedeckel 25 in Fig. 4 gehört zur Variante eines Dispensers mit Solarmodul 19; die einzelnen Solarzellen sind ebenfalls in Fig. 4 eingezeichnet. Auf der oberen Stirnseite dieses Gehäusedeckels 25 befindet sich links ein Lufteinlass 5 mit einem eingelegten Filtersieb 5a sowie rechts eine lichtdurchlässige Abdeckung 33, unter welcher der Schalter 32, Fig. 1, angeordnet ist.

Frontseitig am Gehäusedeckel 25 sind eine Ausblasöffnung 8 und eine Austrittsöffnung 8' vorgesehen. Hinter der schlitzartigen Austrittsöffnung 8' befindet sich ein Schieber 10 der zur Einstellung des Volumenstroms q', zur Anpassung der Grundbeduftung, herangezogen wird.

Die Volumenströme q aus der Umgebungsluft A bzw. die gesättigte Volumenstrom q' und q* mit entsprechenden Atmosphäre A* sind ebenfalls eingezeichnet.

Aus Fig. 1, in Verbindung mit Fig. 4 erkennt man, dass die horizontale Trennfläche 30 den Dispenser in eine obere, und eine untere als Verdunstungs-Raum 20 dienende Kammer aufteilt.

Eine vereinfachte Schaltungsanordnung ist in Fig. 5 dargestellt.

Ein Schallsensor 34 - ein Elektret-Mikrofon - detektiert den Bedarf an "Beduftung". Der eine Ausgang des Schallsensors 34 ist über einen Kondensator C1 an den einen Eingang eines Verstärkers A1 geschaltet, dessen Ausgang ist über eine als Demodulator wirkende Diode D1 zum Eingang eines zweiten Verstärkers A2 geführt. Der Ausgang des Verstärkers A2 ist auf einem Komparator A3 geführt; dessen beiden Eingänge sind über ein Netzwerk R4, R5 zur Einstellung der minimalen Ansprechschwelle und über einen Spannungsteiler R2, R3 geschaltet, welcher die momentane Signaländerung festlegt, verbunden.

Die übrigen Komponenten der Widerstand R1 und der Kondensator C3 sind in konventioneller Weise zugeordnet. Der Widerstand Rl bestimmt die Rückkopplung und damit das Verstärkungsverhältnis welches insgesamt 1:450 beträgt. Der Kondenstor C2 und die weitere Diode D2 sind Bestandteile einer Siebkette und unterdrücken kurzzeitige, ungewollte Signale.

Die vorgängig beschriebene Schaltung des Komparators A3 ermöglicht langsam anschwellende Geräuschpegel zu unterdrücken und erst kräftige akustische Impulse zur Aktivierung des Verdichters heranzuziehen. Die Schaltung ist - empirisch - so ausgelegt, dass das Schliessen einer Türe oder die Betätigung einer WC-Spülung als Nutzsignal gewertet wird und somit der Bedarfsmeldung für die Aktivierung dient.

Nicht dargestellt ist ein notorisch bekannter Mikrokontroller, welcher in üblicherweise ein Signal Us als Steuersignal für die Schaltungsanordnung nach Fig. 5 generiert und welcher durch das Ausgangssignal S des Komparators A3 angesteuert wird.

Die vorgängig beschriebenen Teile sind sowohl in der Variante mit Solarmodul als auch in einer rein batteriegespeisten Variante eines Dispensers vorhanden. Selbstverständlich besitzt die nur batteriegespeiste Variante einen Gehäusedeckel 25 ohne Solarzellen und ebenfalls keinen Speicherkondensator 16.

Demgegenüber kann die Variante mit Solarmodul auf einen lichtsensitiven Schalter 32 verzichten, wenn die Tag-/Nachtschaltung von der Generatorleistung des Solarmoduls 19 abhängig gemacht wird.

Es hat sich gezeigt dass auch die rein durch Batterien gespeiste Variante eines Dispensers im praktischen Einsatz ein Jahr problemlos mit zwei Monozellen arbeitet und die Variante mit Solarmodul sogar auf die Batterien ganz verzichten kann.

Mit einem handelsüblichen Wirkstoff (DRAGOCO, D-37601 Holzminden) und Verdunstungselementen (23, 24) aus notorisch bekanntem Papiervlies von je 24 cm² Schattenfläche hat sich eine mittlere Laufzeit des Verdichters von 3.0s bis 20.05 bei einer Raumtemperatur von 20° C bewährt. Die zeitliche Begrenzung zwischen zwei möglichen Luftwechseln im Verdunstungs-Raum 20 sind auf 4.0 min eingestellt; dieses Intervall genügt einerseits um eine ausreichende Sättigung im Verdunstungs-Raum 20 zu erzielen und andererseits den Anforderungen der Benutzer zu genügen; es ist bedarfsgerecht.

Bewährt hat sich zur Einstellung ein DIP-Switch der mit "Sommer" und "Winter" bezeichnet ist und direkt auf den vorprogrammierten Mikrokontroller wirkt.

Die obige, anhand von zwei praktisch realisierten Ausführungsbeispielen geführte Darstellung lässt sich selbstverständlich erweitern und anpassen auf weitere Ausführungsvarianten.

So kann beispielsweise ein erfindungsgemässer Verdunstungs-Dispenser auch an oder bei einer Türe angeordnet werden und von dieser in einfacher Weise elektromechanisch oder berührungslos aktiviert werden. Im Falle der berührungslosen Schaltung des Ventilators kann durch nebeneinander befindliche Sensoren auch die Wegrichtung des Schliessvorganges der Türe erfasst werden. So kann im Sinne einer Einsparung von Wirkstoff die Einschaltung des Ventilators einzig auf ein Schliessen oder einzig auf ein öffnen der Türe beschränkt werden.

Selbstverständlich kann im Sinne einer Optimierung der Förderleistung das Laufrad des Ventilators durch verbesserte Schaufeln versehen werden, vorzugsweise wird man sogenannte vorwärtsgekrümmte Laufradschaufeln verwenden.

Ebenfalls lässt sich der Ventilator durch andere geeignete Verdichter ersetzen, wie Membranpumpen, Piezo-Förderer etc.

Die Detektion von Schallquellen zur Aktivierung des Ventilators hat sich im bereich von Toiletten als zuverlässig und genügend selektiv erwiesen; Bewegungsmelder beliebiger Art, beispielsweise Infrarot-Melder (PIR) können aber je nach Einsatzort und Einsatzdoktrin günstiger sein.

## Patentansprüche

1. Verfahren zum Betrieb eines Verdunstungs-Dispensers zur Abgabe von desodorierenden und/oder Gerüche neutralisierenden und/oder parfümierenden flüchtigen Wirkstoffen, welche in wenigstens einer Vorratsflasche enthalten sind und über wenigstens ein Verdunstungselement in einem zumindest partiell geschlossenen Raum verdunsten und eine mit dem flüchtigen Wirkstoff angereicherte Atmosphäre bilden, welche sich im Bereich des Sättigungsdampfdruckes der Stoffkomponenten einstellt und wobei diese angereicherte Atmosphäre mittels eines durch einen autonom gespeisten Gleichstrommotor und durch diesen angetriebenen Verdichter mechanisch beschleunigt in die Umgebungsluft verströmt wird, **dadurch gekennzeichnet, dass** der Gleichstrommotor des Verdichters periodisch und/oder bedarfsabhängig an die autonome Stromquelle angeschlossen wird, wobei die mittlere Laufzeit des Verdichters bei jeder Aktivierung auf 3 s bis 20 s eingestellt wird und dass eine Begrenzungszeit zwischen zwei möglichen Luftwechseln im Verdunstungsraum vorgesehen wird, bevor der Verdichter erneut aktivierbar ist, derart, dass sich während dieses Intervalls die mit Wirkstoff angereicherte Atmosphäre im Verdunstungsraum bildet.

2. Verdunstungs-Dispensers zur Durchführung des Verfahrens nach Anspruch 1, zur Abgabe von desodorierenden und/oder Gerüche neutralisierenden und/oder parfümierenden flüchtigen Wirkstoffen, welche in wenigstens einer Vorratsflasche enthalten sind und über wenigstens ein Verdunstungselement in einem zumindest partiell geschlossenen Raum verdunsten und eine mit dem flüchtigen Wirkstoff angereicherte Atmosphäre bilden, welche sich im Bereich des Sättigungsdampfdruckes der Stoffkomponenten einstellt und wobei diese angereicherte Atmosphäre mittels eines durch einen autonom gespeisten Gleichstrommotor und durch diesen angetriebenen Verdichter mechanisch beschleunigt in die Umgebungsluft verströmt, **dadurch gekennzeichnet, dass** der Verdichter (3) ein Zug-/Druck- Ventilator ist, welcher saugseitig eine etwa gleiche Menge (q*) gesättigte Atmosphäre (A*) aus dem Verdunstungs-Raum (20) abführt und in die Umgebungsluft (A) ausbläst wie er druckseitig in den Verdunstungs-Raum (20) fördert, wobei der Zug-/Druck- Ventilator (3) ein einziges Laufrad (11) mit einer radialen Trennwand (13) aufweist, welche in zwei getrennten Kammern (14, 15) drehbar angeordnet ist.

3. Verdunstungs-Dispenser nach Anspruch 2, **dadurch gekennzeichnet, dass** der Verdunstungs-Raum (20) zur Aufnahme von zwei Vorratsflaschen (21, 22) mit Verdunstungselementen (23, 24) Raum bietet, wobei eine der beiden Flaschen (21) im Bereich einer Absaugöffnung (7) des Saugteils des Ventilators (3) plaziert ist.

4. Verdunstungs-Dispenser nach Anspruch 3, **dadurch gekennzeichnet, dass** der Absaugöffnung (7) eine einstellbare Blende (9) vorgelagert ist.

5. Verdunstungs-Dispenser nach Anspruch 1, **dadurch gekennzeichnet, dass** am oberen Teil eines Gerätegehäuses (25) ein Lufteinlass (5) vorgesehen ist.

6. Verdunstungs-Dispenser nach Anspruch 5, **dadurch gekennzeichnet, dass** im Gerätegehäuse (25) eine weitere Austrittsöffnung (8') vorgesehen ist, welche zum Verdunstungs-Raum (20) führt und einen Schieber (10) aufweist.

7. Verdunstungs-Dispenser nach Anspruch 6, **dadurch gekennzeichnet, dass** das Laufrad (11) durch einen Gleichstrommotor (4) angetrieben ist, welcher von einer autonomen Quelle gespeist und die über eine elektronische Schaltung (17) bedarfsorientiert, intermittierend und/oder periodisch zugeschaltet ist.

8. Verdunstungs-Dispenser nach Anspruch, **dadurch gekennzeichnet, dass** die autonome Quelle wenigstens eine Batterie (18) und/oder ein Solarmodul (19) aufweist.

9. Verdunstungs-Dispenser nach Anspruch 7 oder 8, **dadurch gekennzeichnet, dass** ein Schallsensor (34) oder ein Bewegungsmelder vorgesehen ist, welche die Raumbenutzung detektieren und eine elektronische Schaltung (17) und den Ventilator (3) aktivieren.

10. Verdunstungs-Dispenser nach einem der Ansprüche 7 bis 9, **dadurch gekennzeichnet, dass** ein lichtsensitiver Schalter (32) vorgesehen ist, welcher die elektronische Schaltung (17) nachts oder bei abgeschalteter Lichtquelle deaktiviert.

11. Verdunstungs-Dispenser nach einem der Ansprüche 7 bis 9, mit einem Solarmodul (19), **dadurch gekennzeichnet, dass** dieses auf das Lichtspektrum künstlicher Lichtquellen abgestimmt ist und diesem ein Kondensator (16) als Pufferspeicher zugeordnet ist.

12. Verdunstungs-Dispenser nach Anspruch 6 und 9 mit einem Mikrofon, **dadurch gekennzeichnet, dass** diesem ein Frequenz-Bandfilter nachgeschaltet ist, welches auf die charakteristischen Frequenzen der bedarfsorientierten, auslösenden Vorgänge abgestimmt ist und Störgeräusche unterdrückt.

## Claims

1. Method of operating an evaporation dispenser for releasing deodorizing and/or odour-neutralizing and/or perfuming volatile active substances which are contained in at least one storage bottle and evaporate in an at least partially closed space and form an atmosphere which is enriched with the volatile active substance at a pressure in the region of the saturation vapour pressure of the substance constituents, and this enriched atmosphere being made to flow in a mechanically accelerated manner into the ambient air by means of a compressor driven by an autonomously powered direct-current motor, **characterized in that** the direct-current motor of the compressor is connected to the autonomous power source periodically and/or according to requirements, the average running time of the compressor for each activation being set to 3 s to 20 s, and **in that** a limiting time between two possible changes of air in the evaporation space before the compressor can be activated once again is provided in such a way that, during this interval, the atmosphere enriched with active substance is formed in the evaporation space.

2. Evaporation dispenser for carrying out the method according to Claim 1, for releasing deodorizing and/or odour-neutralizing and/or perfuming volatile active substances which are contained in at least one storage bottle and evaporate through an evaporation element in an at least partially closed space and form an atmosphere which is enriched with the volatile active substance at a pressure in the region of the saturation vapour pressure of the substance constituents, and this enriched atmosphere flowing in a mechanically accelerated manner into the ambient air by means of a compressor driven by an autonomously powered direct-current motor, **characterized in that** the compressor (3) is a suction/pressure fan which on the suction side draws off a quantity (q*) of saturated atmosphere (A*) from the evaporation space (20) and discharges it into the ambient air (A) which is approximately equal to the quantity which it supplys on the pressure side to the evaporation space (20), the suction/pressure fan (3) having a single impeller (11) with a radial partition (13), which is arranged rotatably in two separate chambers (14, 15).

3. Evaporation dispenser according to Claim 2,
**characterized in that** the evaporation space (20) has space for holding two storage bottles (21, 22) with evaporating elements (23, 24), one of the two bottles (21) being placed in the region of a suction opening (7) of the suction part of the fan (3).

4. Evaporation dispenser according to Claim 3,
**characterized in that** an adjustable shutter (9) is mounted upstream of the suction opening (7).

5. Evaporation dispenser according to Claim 1,
**characterized in that** an air inlet (5) is provided on the upper part of a housing (25) for the dispenser.

6. Evaporation dispenser according to Claim 5,
**characterized in that** a further outlet opening (8'), which leads to the evaporation space (20) and has a shutter (10), is provided in the housing (25) for the dispenser.

7. Evaporation dispenser according to Claim 6,
**characterized in that** the impeller (11) is driven by a direct-current motor (4), which is powered by an autonomous source and is switched on via an electronic circuit (17) intermittently and/or periodically, according to requirements.

8. Evaporation dispenser according to Claim 7,
**characterized in that** the autonomous source has at least one battery (18) and/or a solar module (19).

9. Evaporation dispenser according to Claim 7 or 8, **characterized in that** a sound sensor (34) and/or a movement detector is provided, which detect use of the space and activate an electronic circuit (17) and the fan (3).

10. Evaporation dispenser according to one of Claims 7 to 9, **characterized in that** a light-sensitive switch (32) is provided, which deactivates the electronic circuit (17) at night-time or when the light source is switched off.

11. Evaporation dispenser according to one of Claims 7 to 9, with a solar module (19), **characterized in that** the latter is set to respond to the light spectrum of artificial light sources and a capacitor (16) is assigned to it as a buffer store.

12. Evaporation dispenser according to Claims 6 and 9 with a microphone, **characterized in that** arranged downstream of the latter is a frequency band filter, which is set to respond to the characteristic frequencies of the requirement-based, triggering events and suppresses random noise.

## Revendications

1. Procédé pour faire fonctionner un diffuseur à évaporation pour la distribution de matières actives volatiles désodorisantes et/ou neutralisant les odeurs et/ou odoriférantes, qui sont contenues dans au moins une bouteille de réserve et s'évaporent dans un espace au moins partiellement fermé par l'intermédiaire d'au moins un élément d'évaporation et forment une atmosphère enrichie par la substance active volatile, qui s'établit dans la région de la pression de vapeur saturante des composants de la matière, cette atmosphère enrichie étant dispersée dans l'air ambiant, accélérée mécaniquement au moyen d'un moteur à courant continu à alimentation autonome et d'un compresseur entraîné par celui-ci, **caractérisé en ce que** le moteur à courant continu du compresseur est raccordé périodiquement et/ou en fonction des besoins à la source de courant autonome, la durée de fonctionnement moyenne du compresseur étant réglée entre 3 s et 20 s à chaque activation, et qu'il est prévu un temps de limitation entre deux renouvellements d'air possibles dans l'espace d'évaporation, avant que le compresseur soit de nouveau activable, si bien que l'atmosphère enrichie de matière active se forme dans cet intervalle dans l'espace d'évaporation.

2. Diffuseur à évaporation pour la mise en oeuvre du procédé suivant la revendication 1, pour la distribution de matières actives volatiles désodorisantes et/ou neutralisant les odeurs et/ou odoriférantes, qui sont contenues dans au moins une bouteille de réserve et s'évaporent dans un espace au moins partiellement fermé par l'intermédiaire d'au moins un élément d'évaporation et forment une atmosphère enrichie par la substance active volatile, qui s'établit dans la région de la pression de vapeur saturante des composants de la matière, cette atmosphère enrichie étant dispersée dans l'air ambiant, accélérée mécaniquement au moyen d'un moteur à courant continu à alimentation autonome et d'un compresseur entraîné par celui-ci, **caractérisé en ce que** le compresseur (3) est un ventilateur tirage/pression, qui évacue du côté aspiration de l'espace d'aspiration (20) et souffle dans l'air ambiant (A) une quantité (q*) d'atmosphère saturée (A*) à peu près égale à celle qu'il refoule du côté refoulement dans l'espace d'évaporation (20), le ventilateur tirage/pression (3) présentant une roue unique (11) comportant une cloison radiale (13), disposée tournante dans deux compartiments séparés (14, 15).

3. Diffuseur à évaporation suivant la revendication 2,
**caractérisé en ce que** l'espace d'évaporation (20) offre un espace pour recevoir deux bouteilles de réserve (21, 22) munies d'éléments d'évaporation (23, 24), l'une des deux bouteilles (21) étant placée dans la zone d'une ouverture d'aspiration (7) de la partie aspiration du ventilateur (3).

4. Diffuseur à évaporation suivant la revendication 3,
**caractérisé en ce qu'**un diaphragme réglable (9) est placé devant l'ouverture d'aspiration (7).

5. Diffuseur à évaporation suivant la revendication 1,
**caractérisé en ce qu'**une admission d'air (5) est prévue sur la partie supérieure d'un carter d'appareil (25).

6. Diffuseur à évaporation suivant la revendication 5,
**caractérisé en ce qu'**une autre ouverture d'échappement (8'), prévue dans le carter (25) de l'appareil, mène à l'espace d'évaporation (20) et présente un registre (10).

7. Diffuseur à évaporation suivant la revendication 6,
**caractérisé en ce que** la roue (11) est entraînée par un moteur à courant continu (4), qui est alimenté par une source de courant autonome et est commuté par intermittence et/ou périodiquement, en fonction des besoins, par un circuit (17) électronique.

8. Diffuseur à évaporation suivant la revendication 7,
**caractérisé en ce que** la source autonome présente au moins une pile (18) et/ou un module solaire (19).

9. Diffuseur à évaporation suivant l'une des revendications 7 et 8, **caractérisé en ce qu'**est prévu un capteur acoustique (34) ou un détecteur de déplacement, qui détectent l'occupation des locaux et activent un circuit électronique (17) et le ventilateur (3).

10. Diffuseur à évaporation suivant l'une des revendications 7 à 9, **caractérisé en ce qu'**est prévu un interrupteur photosensible (32), qui désactive le circuit électronique (17) de nuit ou en cas de source lumineuse déconnectée.

11. Diffuseur à évaporation suivant l'une des revendications 7 à 9 comprenant un module solaire (19), **caractérisé en ce que** celui-ci est adapté au spectre de sources de lumière artificielle et qu'un condensateur (16) est associé à celui-ci en tant qu'accumulateur tampon.

12. Diffuseur à évaporation suivant les revendications 6 et 9 comprenant un microphone, **caractérisé en ce qu'**un filtre passe-bande, monté en aval de celui-ci, est adapté aux fréquences caractéristiques des processus de déclenchement orientés sur les besoins, et supprime des bruits parasites.
